# EUROPÄISCHE PATENTSCHRIFT

(11) **EP 2 049 650 B1**
(45) Veröffentlichungstag und Bekanntmachung des Hinweises auf die Patenterteilung: **06.01.2016**
(21) Anmeldenummer: 07722501.9
(22) Anmeldetag: 01.06.2007
(51) Int. Cl.: C12M 1/26, C12M 1/34, C12Q 1/06, B01D 67/00

(54) **MIKROMECHANISCHER FILTER FÜR MIKROPARTIKEL, INSBESONDERE FÜR PATHOGENE BAKTERIEN UND VIREN, SOWIE VERFAHREN ZU SEINER HERSTELLUNG**
MICROMECHANICAL FILTER FOR MICROPARTICLES, IN PARTICULAR FOR PATHOGENIC BACTERIA AND VIRUSES, AND ALSO PROCESS FOR PRODUCTION THEREOF
FILTRE MICROMÉCANIQUE POUR MICROPARTICULES, EN PARTICULIER POUR DES BACTÉRIES PATHOGÈNES ET DES VIRUS, ET SON PROCÉDÉ DE FABRICATION

(30) Priorität: 06.06.2006 DE 102006026559
(43) Veröffentlichungstag der Anmeldung: 22.04.2009
(73) Patentinhaber: Speetect GmbH, 82024 Taufkirchen (DE)
(72) Erfinder: FRIEDBERGER, Alois, 85667 Oberpframmern (DE); MÜLLER, Gerhard, 85567 Grafing (DE)
(74) Vertreter: Krebs, Jörg
(86) Internationale Anmeldenummer: PCT/DE2007/000987
(87) Internationale Veröffentlichungsnummer: WO 2007/140752

(56) Entgegenhaltungen:
- EP-A- 0 148 290
- EP-A1- 0 317 399
- JP-A- 2 180 624
- US-A1- 2004 002 126
- US-A1- 2005 092 181

## Beschreibung

Die Erfindung betrifft einen mikromechanischen Filter für Mikropartikel gemäß dem Oberbegriff von Patentanspruch 1, sowie ein Verfahren zur Herstellung eines solchen.

Mikromechanische Filter für Mikropartikel, wie beispielsweise pathogene Bakterien, sonstige Keime, Viren, usw., sind insbesondere im Bereich der Trinkwasserversorgung einsetzbar, um die Trinkwassernetze gegen Verschmutzungen zu schützen. Dabei müssen die Versorgungsnetze zum einen vor dem Eindringen unerwünschter Partikel geschützt werden, andererseits ist es notwendig, vorhandene Partikel zu detektieren und ggf. den Verschmutzungsgrad oder eine Anzahl von Keimen bzw. Bakterien oder auch Viren festzustellen. Auch in der Luft müssen Krankheitserreger wie beispielsweise pathogene Keime, Bakterien und Viren zuverlässig festgestellt werden. Dabei ist insbesondere auch die Detektion biologischer Kampfstoffe in Flüssigkeiten und Gasen von Bedeutung.

Die Druckschrift DE 101 34 860 A1 beschreibt eine Vorrichtung und ein Verfahren zum Erfassen immunogener Partikel mit einem Filterteil zum Zurückhalten der immunogenen Partikel und mit einem Sensorelement zum Empfangen eines durch im Filtermaterial befindliche immunogene Partikel erzeugten Signals. Dabei ist der Filterteil in Form eines porösen Filters ausgebildet, dessen Porengröße kleiner als der Durchmesser der nachzuweisenden immunogenen Partikel ist, so dass eine Anreicherung der Partikel in Form einer Oberflächenfiltration auf dem Filter erfolgt. Das Filterelement ist in Form eines auswechselbaren Filterteils ausgebildet, welches vor einer jeweiligen Messung in die Vorrichtung neu eingelegt werden soll. Der immunologische Nachweis der als Filterkuchen auf der Oberfläche des Filterteils angesammelten immunogenen Partikel soll durch Einbringen und Inkubieren von spezifischen, mit einer signalerzeugenden Substanz markierten Antikörpern und Detektieren eines dadurch von der Filterteiloberfläche ausgehenden Signals in der Nähe des Filterteils erfolgen. Der immunologische Nachweis direkt auf der Oberfläche des Filterteils erfolgt, indem ein für die nachzuweisenden immunogenen Partikel spezifischer Antikörper durch den Filterkuchen und das Filterteil geleitet wird. Sind auf der Filteroberfläche zu dem spezifischen Antikörper passende immunogene Partikel vorhanden, geschieht eine katalytische Umsetzung durch Chemilumineszenz unter Erzeugung von Licht, welches mittels eines hinter einem lichtdurchlässigen Fenster befindlichen Fotodetektor erfaßt und zum Nachweis der immunogenen Partikel genutzt wird.

Das US-Patent 5, 258,285 zeigt ein Verfahren zur Detektion einer Bakterienkonzentration in einer Probe, bei dem Zellpopulationen auf der Oberfläche eines bewegbaren Filtermaterials konzentriert werden. Zur Messung wird das Filtermaterial, das die konzentrierten Bakterienzellen enthält, zu einer Extraktionskammer bewegt.

Die Druckschrift EP 0 612 850 B1 beschreibt ein Verfahren zur Bestimmung der Anzahl von Mikroorganismen in einer Probenlösung, bei dem die Probenlösung durch eine Filtrationsmembran filtriert wird, um Mikroben auf dieser einzufangen. Die mikrobenhaltige Membran wird mit einer Lösung eines ATP-Extraktionsreagenz und anschließend mit einer Lösung eines Lumineszenz induzierenden Reagenz besprüht, um schließlich den Lumineszenzgrad zu bestimmen.

Die US-A-2005/0092181 beschreibt Filtereinrichtungen, welche dazu dienen, pathogene Mikroorganismen oder brennbare Restbestandteile von Dieselabgasen aufzufangen und zu vernichten, bei welchen mindestens ein poröses Filterelement vorgesehen ist, das einer elektrischen Widerstandsheizung unterzogen werden kann, so dass die pathogenen Mikroorganismen unschädlich gemacht und/oder die besagten verbrennbaren Gaspartikel vernichtet werden. Das verwendete Filter ist ein typisches Volumenfilter, welches beispielsweise aus einer Anzahl von übereinandergestapelten porösen Schichten, insbesondere eines Schaummaterials bestehen kann, wobei davon ausgegangen wird, dass die aufzufangenden Partikel aufgrund der labyrinthartigen Natur des porösen Materials irgendwo in dessen Tiefe gefangen werden. Bei dieser bekannten Filtereinrichtung gibt es weder ein in Form einer perforierten Membran vorgesehenes Oberflächenfilter, noch besteht die Möglichkeit, die Mikropartikel von dem Filterelement zu entfernen und einer Detektoreinheit zur Detektion zuzuführen. Es können lediglich die im Volumenfilter festgehaltenen Partikel unschädlich gemacht werden.

Die EP-A-0148290 beschreibt eine Filtereinrichtung, die dazu vorgesehen ist, in einer Flüssigkeit vorhandene Partikel, insbesondere Mikroorganismen zu konzentrieren und einer Analyse zugänglich zu machen. Dazu ist ein Filter in Form eines beweglichen Bandes vorgesehen, das an zwei Filterhaltern vorbei beweglich ist. An dem ersten Filterhalter wird das Filterband von der zu untersuchenden Probeflüssigkeit durchströmt, wobei die in der Flüssigkeit vorhandenen Partikel oder Mikroorganismen im Material des Filterbandes konzentriert werden, dann wird der die konzentrierten Partikel enthaltende Bereich des Filterbandes zum zweiten Filterhalter weitertransportiert, wo die konzentrierten Partikel an eine den zweiten Filterhalter 7 durchströmende Flüssigkeit abgegeben und diese dann einer Analyse, beispielsweise einer Chemolumineszenzanalyse unterzogen wird.

Aus der US-A-2004/00021226 sind Verfahren und Vorrichtung zur Detektion von in Luft vorhandenen Mikroorganismen bekannt, bei denen an in einer trommelförmigen Kammer rotierenden Filterscheibe aus der diese durchströmenden Luft aufgenommene Mikroorganismen bei der Drehung der Filterscheibe an eine in im unteren Bereich der Trommel vorhandene Flüssigkeit 5 abgegeben und mit dieser dann einer Erfassung oder Analyse zugeführt wird. Das Filter besteht aus einer scheibenförmigen Filtermembran, welche zwischen zwei perforierten scheibenförmigen Haltern angeordnet ist.

Bei den bekannten Detektions- und Filterverfahren besteht das Bedürfnis, die Anreicherung von Bakterien zu erhöhen, um das Detektionslimit zu verbessern. Weiterhin sollen die eingesetzten Filter möglichst oft wieder verwendet werden können. Im Bereich der Detektionsverfahren soll eine höhere Sensitivität erzielt werden. Vor allem als Volumenfilter ausgestaltete konventionelle Filter haben den Nachteil einer schnellen Verschmutzung, und die bisher bekannten Mikrofilter zeigen in vielen Fällen eine beschränkte mechanische Stabilität.

Es ist die Aufgabe der vorliegenden Erfindung, einen Filter für Mikropartikel und insbesondere für Bakterien und Viren zu schaffen, der es gestattet Mikropartikel wirksam an seiner Oberfläche anzureichern und der bei einer längeren Lebensdauer ohne großen Aufwand wiederholt verwendbar ist, so dass zahlreiche aufeinanderfolgende Messungen durchgeführt werden können.

Diese Aufgabe wird gelöst durch den mikromechanischen Filter gemäß Patentanspruch 1. Ein Verfahren zur Herstellung eines solchen mikromechanischen Filters ist Gegenstand des Patentanspruchs 13.

Weitere vorteilhafte Merkmale, Aspekte und Details der Erfindung ergeben sich aus den abhängigen Ansprüchen, der Beschreibung und den Zeichnungen.

Der erfindungsgemäße mikromechanische Filter für Mikropartikel ist insbesondere zur Filterung pathogener Bakterien und Viren geeignet und umfasst ein Substrat sowie eine fest mit dem Substrat verbundene, perforierte Membran zum Herausfiltern von Mikropartikeln aus einem Medium beim Durchströmen der Membran, und weiterhin eine Einrichtung zum Entfernen der herausgefilterten Mikropartikel von der Oberfläche der Membran. Die Einrichtung zum Entfernen der herausgefilterten Mikropartikel von der Oberfläche der Membran umfasst eine Aktorstruktur, die an der Membran befestigt ist, um die Membran zu verformen.

Der erfindungsgemäße mikromechanische Filter hat eine erhöhte Lebensdauer und eine hohe mechanische Stabilität. Er ist darüber hinaus wieder verwendbar und ermöglicht insbesondere auch eine Detektion von Bakterien und anderen Keimen oder auch Viren mit hoher Sensitivität. Der mikromechanische Filter dient nicht nur zum Herausfiltern und Anreichern von Keimen bzw. Mikropartikeln, die in Wasser oder anderen Flüssigkeiten vorhanden sind, sondern auch zum Herausfiltern und Anreichern von Mikropartikeln und Keimen, die in der Luft bzw. in einem Gas vorhanden sind. Damit können z.B. Krankheitserreger oder biologische Kampfstoffe detektiert werden.

Vorteilhafterweise ist die Aktorstruktur derart ausgestaltet, dass sie in der Membran Wellenbewegungen in Form von Oberflächenwellen erzeugt.

Die Aktorstruktur kann durch ein oder mehrere FPW-Strukturen gebildet sein.

Durch das Aufbringen von Aktorstrukturen, die z.B. FPW-Strukturen (flexural plate wave) sind, auf die Chipoberfläche bzw. Membranoberfläche, kann im Betrieb an der Oberfläche der mit Mikroporen ausgestalteten Membran eine Agitation erzeugt werden, durch die vorhandene Partikel oder Keime von der Filteroberfläche gelöst oder auch wegtransportiert werden. D.h., die Aktor- bzw. FPW-Struktur erzeugt Wellen in der Membran, die einen Abtransport der Verschmutzung bzw. der vorhandenen Partikel bewirken. Andererseits können durch die Agitation der Membran auch biochemische Vorgänge an der Filteroberfläche beschleunigt werden.

Vorteilhaft umfasst die Einrichtung zum Entfernen der Mikropartikel eine Heizeinrichtung zum Heizen der Membran, um die an der Oberfläche der Membran befindlichen Mikropartikel zu verbrennen.

Dadurch wird es möglich, den Mikrofilter durch Freibrennen zu reinigen, wobei die Membran beispielsweise durch einen Stromfluss so weit erhitzbar ist, dass alle brennbaren Materialien, die sich an der Oberfläche der Membran angesammelt haben, entfernt werden. Dabei ist die Membran beispielsweise auf 700 °C und mehr erhitzbar, bevorzugt auf ca. 1.000 °C und insbesondere bevorzugt auf ca. 1.200 °C. Dabei eignet sich besonders eine Membran, die aus Siliziumkarbid bzw. SiC hergestellt ist.

Die Heizeinrichtung ist beispielsweise durch elektrische Kontakte gebildet, die derart ausgestaltet sind, dass beim Anschluss einer Spannungsquelle ein Heizstrom durch die Membran fließt. Dies hat den besonderen Vorteil eines geringen konstruktiven Aufwandes, wobei neben den Kontakten keine zusätzlichen Bauteile benötigt werden.

Es ist jedoch auch vorteilhaft, die Heizeinrichtung durch einen Heizmäander zu bilden, der thermisch an die Membran gekoppelt ist.

Durch die Heizeinrichtung ist es auch möglich, die Membran zu desinfizieren bzw. zu sterilisieren, so dass der Filter wieder verwendbar ist und zahlreiche aufeinander folgende Messungen durchgeführt werden können.

Vorteilhaft umfasst die Einrichtung zum Entfernen der Mikropartikel eine Mikropumpe und/oder einen Mikroinjektor, der eine Strömung parallel zur Oberfläche der Membran erzeugt, welche die Bakterien oder Partikel von der Membran löst. Dadurch können die Keime bzw. Mikropartikel vom Mikrofilter entfernt und weiter transportiert werden, beispielsweise zu einer Detektionseinheit. Dabei bietet die Mikrofilteroberfläche den besonderen Vorteil, dass sich z.B. Bakterien nach der Anreicherung sehr einfach wieder von der Mikrofilteroberfläche entfernen lassen. Bei normalen Filtern bzw. Volumenfiltern können nur ca. 50% wieder entfernt werden. Bevorzugt ist der mikromechanische Filter in ein mikrofluidisches System eingebaut.

Vorteilhafterweise umfasst der mikromechanische Filter eine Vorrichtung zur Vervielfältigung von Bakterien, die von der Oberfläche der Membran entfernt wurden. Die Vorrichtung kann beispielsweise ein Mikroreaktor oder ähnliches sein, der zur Durchführung einer Polymerase-Kettenreaktion bzw. PCR (polymerase chain reaction) ausgestaltet ist, d.h. eine Vervielfältigung der DNA durchführt. Dadurch wird die Genauigkeit und Empfindlichkeit der Messung erheblich gesteigert.

Bevorzugt umfasst die Vorrichtung eine Detektoreinheit zur Detektion der von der Oberfläche der Membran entfernten und/oder der vervielfältigten Keime. Dadurch kann beispielsweise die Bakterienart festgestellt werden, und es können Sporen, Viren und andere Mikropartikel festgestellt werden. Insbesondere in Kombination mit einer Vervielfältigung von Bakterien ergibt sich eine besonders große Messgenauigkeit bei der Detektion.

Bevorzugt ist die Membran aus einkristallinem Silizium gebildet, wobei bevorzugt auch das Substrat aus einkristallinem Silizium gebildet ist. Dadurch ergibt sich eine besonders große mechanische Stabilität, insbesondere dadurch, dass nicht nur der Träger der Membran, d.h. das Substrat, sondern auch das Membranmaterial selbst aus einkristallinem Silizium gebildet ist.

Vorteilhafterweise ist die Membran und/oder das Substrat aus Siliziumkarbid gebildet. Dadurch ergibt sich eine noch größere mechanische Stabilität sowie eine höhere chemische und thermische Beständigkeit. Dabei kann das Siliziumkarbid ein- oder polykristallin ausgebildet sein.

Vorteilhafterweise ist der mikromechanische Filter aus einem Metall hergestellt, das oxidationsbeständig beschichtet ist. Auch durch diese Maßnahme wird eine hohe mechanische Stabilität bei einer hohen chemischen und thermischen Beständigkeit erreicht.

Gemäß einem anderen Aspekt der Erfindung wird ein Verfahren zur Herstellung eines mikromechanischen Filters angegeben, bei dem ein Teil eines Substrats porosiziert wird, um eine mit Löchern versehene Schicht zu bilden, und ein anderer Teil des Substrats entfernt wird, so dass aus dem Substrat eine Membran herausgearbeitet wird, wobei die Membran durch die mit Löchern versehene Schicht gebildet wird, und weiterhin eine Einrichtung zum Entfernen von Ablagerungen von einer Oberfläche der Membran ausgestaltet wird, welche eine Aktorstruktur umfasst, die an der Membran befestigt ist, um die Membran zu verformen.

Bevorzugt wird zunächst das Porosizieren des Substrats von dessen Oberseite her bis zu einer definierten Tiefe durchgeführt, und anschließend wird der andere Teil des Substrats von dessen Unterseite her zumindest teilweise entfernt, so dass die poröse Schicht eine Membran mit durchgehenden Löchern bildet.

Alternativ dazu umfasst das Substrat eine untere Substratschicht mit einem darüber angeordneten SOI-Wafer (SOI = silicon on insulator), wobei ein Teil der unteren Substratschicht durch Ätzen entfernt wird und wobei die Isolatorschicht des SOI-Wafers als Ätzstopp verwendet wird.

Vorteilhaft wird nach dem Ätzen der unteren Substratschicht die Isolatorschicht des SOI-Wafers entfernt, und anschließend wird die Siliziumschicht des SOI-Wafers porosiziert, um die mit durchgehenden Löchern versehene Membran zu bilden.

Nachfolgend wird die Erfindung beispielhaft anhand der Zeichnungen beschrieben, in denen
**Fig. 1** einen mikromechanischen Filter mit einer Heizeinrichtung zum Entfernen von Mikropartikeln gemäß einer ersten bevorzugten Ausführungsform als Draufsicht und als Schnittansicht zeigt;
**Fig. 2** eine Membran mit einem Heizmäander als Heizeinrichtung für einen mikromechanischen Filter gemäß einer zweiten bevorzugten Ausführungsform schematisch als Draufsicht zeigt;
**Fig. 3** einen mikromechanischen Filter mit einem Mikroinjektor zum Entfernen von Mikropartikeln gemäß einer dritten bevorzugten Ausführungsform der Erfindung schematisch zeigt;
**Fig. 4** einen mikromechanischen Filter mit einer Aktorstruktur zum Entfernen von Partikeln gemäß einer vierten bevorzugten Ausführungsform als Draufsicht schematisch zeigt;
**Fig. 5a** **und b** ein Substrat zur Herstellung eines erfindungsgemäßen mikromechanischen Filters in zwei unterschiedlichen Stufen der Herstellung zeigt; und
**Fig. 6a** **bis c** ein Substrat zur Herstellung eines mikromechanischen Filters in drei unterschiedlichen Herstellungsstufen gemäß einem anderen bevorzugten Herstellungsverfahren schematisch zeigt.

Vorteile und Merkmale, die in Zusammenhang mit dem mikromechanischen Filter beschrieben sind, gelten auch für das erfindungsgemäße Verfahren, und umgekehrt. Elemente mit im Wesentlichen gleichen Eigenschaften oder Funktionen sind in den Figuren mit denselben Bezugszeichen gekennzeichnet.

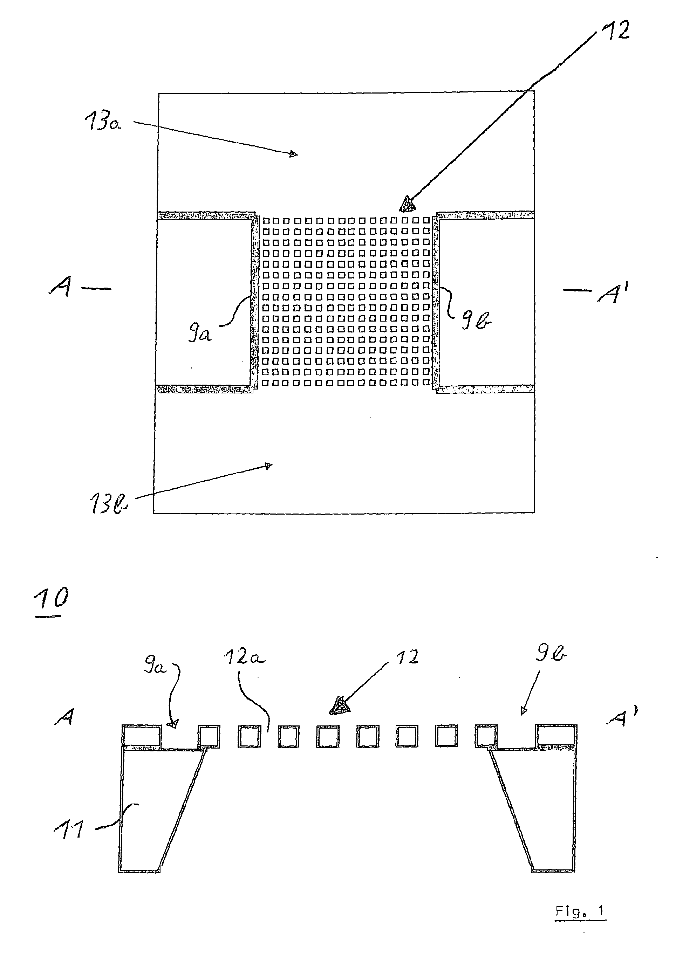

Fig. 1 zeigt einen mikromechanischen Filter 10 als erste bevorzugte Ausführungsform der Erfindung in einer Draufsicht und als Schnittansicht entlang der Linie A-A'. Der mikromechanische Filter 10 hat in seinem unteren Bereich ein strukturiertes Substrat 11, das eine perforierte Membran 12 trägt. Die Membran 12 ist mit durchgehenden Löchern 12a versehen und dient zum Herausfiltern von Mikropartikeln aus einem Medium beim Durchströmen der Membran 12. An der Oberseite der Membran 12 befindet sich eine erste Kontaktfläche 13a und eine zweite Kontaktfläche 13b zum elektrischen Anschluss einer Spannungsversorgung. Die Spannungsversorgung bewirkt einen elektrischen Strom zwischen den Kontaktflächen 13a und 13b durch die perforierte Membran 12, so dass diese aufgrund des Stromflusses geheizt wird. Bei einer Heiztemperatur von beispielsweise 700 °C bis 1.000 °C erfolgt ein Verbrennen der herausgefilterten Mikropartikel, die sich an der Oberfläche der Membran 12 befinden. D.h., die beiden Kontaktflächen 13a, 13b bilden eine Einrichtung zum Entfernen der herausgefilterten Mikropartikel von der Oberfläche der Membran 12.

Die perforierte Membran 12 ist entlang der beiden Linien 9a, 9b derart strukturiert, dass dort der Stromfluss unterbrochen ist und der elektrische Strom über die perforierte Membran 12 fließt, wenn diese an den Kontaktflächen 13a, 13b kontaktiert wird.

Das Substrat 11 besteht aus einkristallinem Silizium. Um eine höhere mechanische Stabilität zu erreichen, besteht auch die mit Mikroporen versehene Membran 12 aus einkristallinem Silizium. Es ist aber auch möglich, anderen Materialien zu verwenden, beispielsweise Siliziumnitrit (Si₃N₄) als Membranmaterial. In Fällen, in denen eine besonders hohe mechanische Stabilität sowie eine besonders hohe chemische und thermische Beständigkeit erforderlich ist, eignet sich insbesondere Siliziumkarbid (SiC) als Material für die Membran 12 und vorzugsweise zusätzlich auch als Material für das Substrat 11, das als Träger für die Membran 12 dient. Dabei kann sowohl einkristallines als auch polykristallines SiC verwendet werden.

Je nach dem Anwendungsgebiet und Einsatzbereich des mikromechanischen Filters 10 eignet sich beispielsweise auch ein oxidationsbeständig beschichtetes Metall als Material für den Filter 12 bzw. Mikrofilter.

Die durchgehenden Löcher 12a der Membran 12 oder Poren haben in der bevorzugten Ausführungsform einen Durchmesser von 450 nm. Je nach Anwendungsgebiet können sie aber auch andere Durchmesser aufweisen, die geeignet sind, Mikropartikel in Form von Bakterien, Viren, Keimen, usw. an der Oberfläche der Membran 12 zurückzuhalten, wenn ein flüssiges oder gasförmiges Medium die Membran 12 durch die Löcher 12a durchströmt.

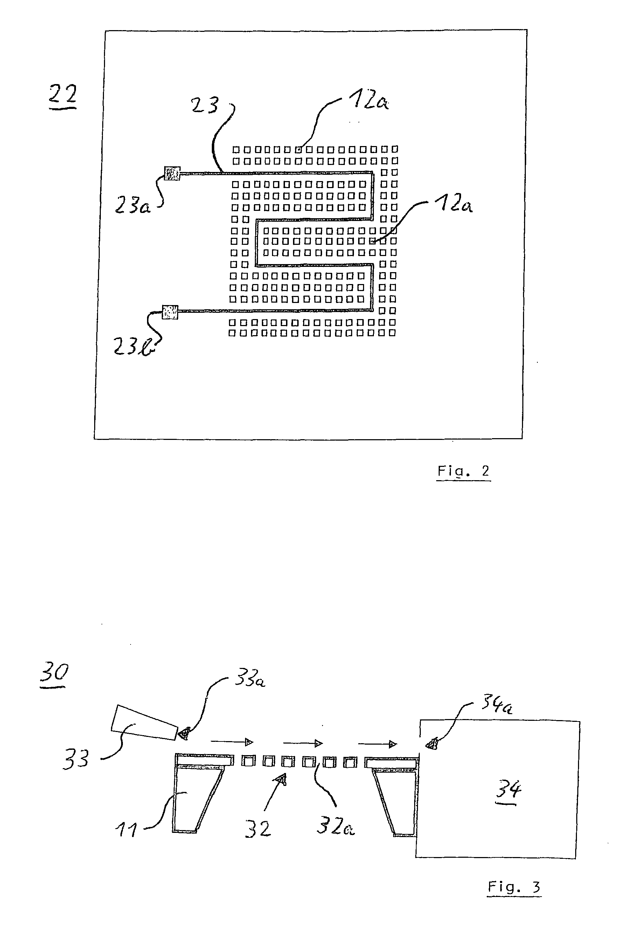

In Fig. 2 ist eine alternativ ausgestaltete Membran 22 als Draufsicht dargestellt, gemäß einer zweiten bevorzugten Ausführungsform der Erfindung. Dabei befindet sich im Bereich der durchgehenden Löcher 12a der Membran 22 ein mäanderförmiges Heizelement 23, das auf die Membran 22 aufgebracht ist und an seinen beiden Enden jeweils eine Kontaktfläche 23a, 23b zum elektrischen Anschluss einer Spannungsversorgung aufweist. Ebenso wie bei der in Fig. 1 dargestellten ersten bevorzugten Ausführungsform werden auch in diesem Fall Rückstände bzw. Mikropartikel, die sich an der Oberfläche der Membran 22 befinden, thermisch bzw. durch Erhitzen der Membran 22 entfernt, d.h. die Rückstände oder Mikropartikel werden verbrannt.

Die übrigen Elemente und Merkmale der in Fig. 2 gezeigten Ausführungsform sind wie oben unter Bezugnahme auf Fig. 1 beschrieben.

Fig. 3 zeigt einen mikromechanischen Filter 30 gemäß einer dritten bevorzugten Ausführungsform der Erfindung. Der mikromechanische Filter 30 hat ein als Träger ausgestaltetes strukturiertes Substrat 11, auf dem eine perforierte Membran 32 gelagert ist, die mit durchgehenden Löchern 32a versehen ist. Die Membran 32 ist fest mit dem darunter liegenden Substrat 11 verbunden. Seitlich an der Membran 32 ist ein Mikroinjektor 33 vorgesehen, der einen Flüssigkeits- oder Gasstrom entlang der Oberfläche der Membran 32 bzw. parallel dazu erzeugt, um dort befindliche Mikropartikel zu entfernen, die als Rückstände nach dem Filtervorgang auf der Membran 32 abgelagert sind. Zu diesem Zweck ist eine Öffnung 33a des Mikroinjektors 33 als Düse ausgestaltet, die auf die Oberfläche der Membran 32 im Bereich der durchgehenden Löcher 32a gerichtet ist. Der Mikroinjektor 33 umfasst eine Mikropumpe, um ein flüssiges oder gasförmiges Medium zum Spülen der Membranoberfläche durch die düsenförmige Öffnung 33a zu pumpen.

An der dem Mikroinjektor 33 gegenüber liegenden Seite der Membran 32 ist ein Mikroreaktor 34 mit einer Detektionseinheit vorgesehen. Der Mikroreaktor 34 umfasst eine Eingangsöffnung 34a, die zur Aufnahme der herausgefilterten und von der Oberfläche der Membran 32 entfernten Mikropartikel in dem Mikroreaktor 34 dient. D.h., dass Keime an der Membranoberfläche nach der Anreicherung durch das dargestellte mikrofluidische System vom Mikrofilter entfernt und weiter transportiert werden, beispielsweise zu einer Detektionseinheit und/oder in einen Mikroreaktor oder ähnliches. In dem hier dargestellten Fall erfolgt im Mikroreaktor 34 eine PCR (polymerase chain reaction), d.h. eine Vervielfältigung der DNA. Dadurch kann beispielsweise eine Bakterienart festgestellt werden. Die Detektion ist aber auch für Sporen, Viren, usw. geeignet.

Die Merkmale und Eigenschaften des Substrats 11 und der Membran 32a entsprechen im Wesentlichen den oben unter Bezugnahme auf die Figuren 1 und 2 diskutierten Merkmalen, wobei jedoch zum Entfernen der Mikropartikel ein Mikroinjektor anstelle einer Heizeinrichtung vorgesehen ist.

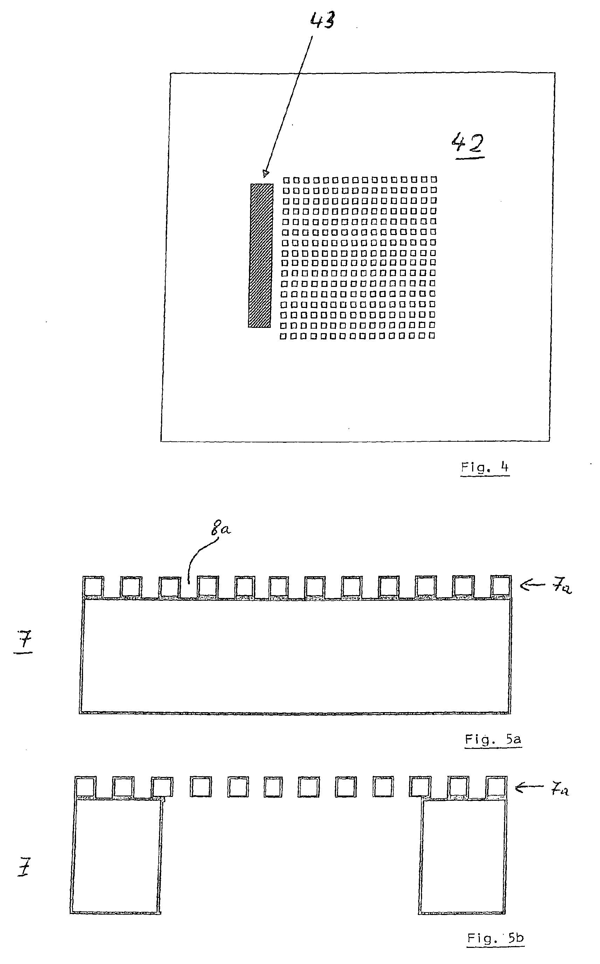

In Fig. 4 ist eine Membran 42 eines Filters gemäß einer vierten bevorzugten Ausführungsform der Erfindung dargestellt. Dabei ist an der Membran 42 eine Aktorstruktur 43 zur Anregung von Oberflächenwellen im Bereich der perforierten Membran 42 angeordnet. Das darunter gelegene Substrat ist wie bei den anderen bereits diskutierten Ausführungsformen ausgestaltet.

Die Aktorstruktur 43 umfasst beispielsweise ein oder mehrere FPW-Strukturen (flexural plate wave), die auf einer Chip- bzw. Membranoberfläche angeordnet sind, um an deren Oberfläche eine Agitation zu erzeugen. Diese Agitation dient der Beschleunigung biochemischer Vorgänge an der Membranoberfläche und/oder dem Transport der Mikropartikel bzw. Keime, die auf der Filteroberfläche als Rückstände gelagert sind, von der Filteroberfläche weg.

Ein Verfahren zur Herstellung des mikromechanischen Filters wird nachfolgend anhand der Figuren 5a und 5b beschrieben.

Zunächst wird ein bereitgestelltes Substrat 7, das z.B. aus Silizium gefertigt ist, ausgehend von dessen Oberfläche porosiziert, so dass es mit dünnen Kanälen oder Löchern 8a durchzogen wird (Fig. 5a). Dabei wird die Porosität bzw. Stärke der mit Kanälen oder Löchern 8a versehenen Schicht 7a des Substrats 7 von der Dotierung des Substrats bestimmt, sowie von der Stromdichte und Zusammensetzung des verwendeten Elektrolyten. Dabei lässt sich der Prozess beispielsweise auch durch elektrochemisches Ätzen unter Lichtbestrahlung weiter anpassen. Sobald die gewünschte Membrandicke erreicht ist, die der Dicke der Schicht 7a entspricht, wird der Vorgang des Porosizierens beendet.

In einem zentralen Bereich der späteren Membran bzw. der perforierten Schicht 7a wird das unterhalb der porosizierten Schicht 7a gelegene Substrat von unten entfernt (Fig. 5b). Das Entfernen des Substratbereichs erfolgt beispielsweise durch konventionelles Trockenätzen. Die einzelnen hier beschriebenen Verfahrensschritte können auch in anderer Reihenfolge durchgeführt werden.

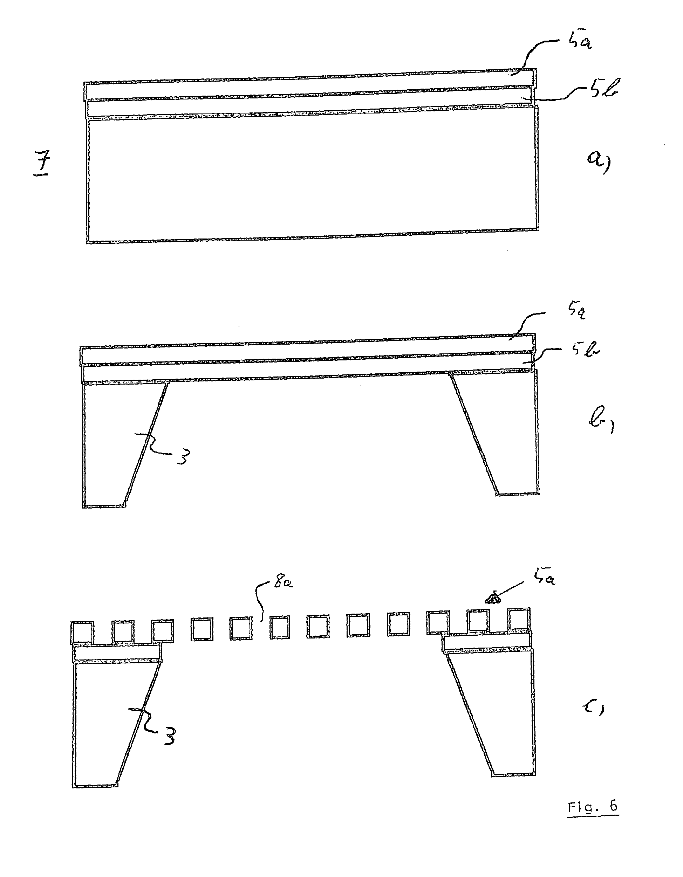

Eine weitere mögliche Ausgestaltung des erfindungsgemäßen Verfahrens wird nun anhand der Figuren 6a bis c beschrieben.

Mit diesem Verfahren lässt sich die Membran mit einer noch exakter definierten Dicke herstellen. Dabei wird ein SOI (silicon on insulator) -Wafer verwendet. Dadurch ist die Dicke der späteren Membran bereits zu Beginn exakt durch die Dicke der obersten Schicht 5a festgelegt (siehe Fig. 6a).

Nun erfolgt das Ätzen des Substrats 7 von dessen Rückseite her. Dabei dient die Isolatorschicht 5b des SOI-Wafers als Ätzstopp. Auf diese Weise wird aus dem Substrat 7 der Träger 3 für die spätere brückenartige Membranstruktur herausgearbeitet (siehe Fig. 6b).

Nun erfolgt das Entfernen des von unten her freigelegten Bereichs der Isolatorschicht 5b durch Ätzen. Schließlich werden in der oberen Schicht 5a des SOI-Wafers, die nun als dünne Membran ausgestaltet ist, die durchgehenden Löcher 8a hergestellt, was im vorliegenden Fall durch elektrochemisches Ätzen erfolgt (Fig. 6c). Besonders bevorzugte Anwendungsgebiete des erfindungsgemäßen mikromechanischen Filters sind beispielsweise die Trinkwasseranalyse, die Analyse von sonstigen flüssigen Medien wie z.B. Blut, die Filterung und Analyse von Luft, die Detektion und Filterung von Krankheitserregern, biologischen Kampfstoffen, o.ä..

## Patentansprüche

1. Mikromechanischer Filter für Mikropartikel, insbesondere für pathogene Bakterien und Viren, mit
einem Substrat (11), und
einer fest mit dem Substrat (11) verbundenen perforierten Membran (12; 22; 32; 42), zum Herausfiltern von Mikropartikeln aus einem Medium beim Durchströmen der Membran (12; 22; 32; 42) **gekennzeichnet durch**
eine Einrichtung (13a, 13b; 23; 33; 43) zum Entfernen der herausgefilterten Mikropartikel von der Oberfläche der Membran (12; 22; 32; 42), welche eine Aktorstruktur (43) umfasst, die an der Membran (42) befestigt ist, um die Membran (42) zu verformen.

2. Mikromechanischer Filter nach Anspruch 1, **dadurch gekennzeichnet, dass** die Aktorstruktur (43) derart ausgestaltet ist, dass sie in der Membran (42) Wellenbewegungen in Form von Oberflächenwellen erzeugt.

3. Mikromechanischer Filter nach Anspruch 1 oder 2, **dadurch gekennzeichnet, dass** die Aktorstruktur (43) durch ein oder mehrere FPW-Strukturen gebildet ist.

4. Mikromechanischer Filter nach Anspruch 1, 2 oder 3, **dadurch gekennzeichnet, dass** die Einrichtung zum Entfernen der Mikropartikel eine Heizeinrichtung (13a, 13b; 23) zum Heizen der Membran (12; 22) umfasst, um die an der Oberfläche der Membran (12; 22) befindlichen Mikropartikel zu verbrennen.

5. Mikromechanischer Filter nach Anspruch 4, **dadurch gekennzeichnet, dass** die Heizeinrichtung durch elektrische Kontakte (13a, 13b) gebildet wird, die derart ausgestaltet sind, dass beim Anschluss einer Spannungsquelle ein Heizstrom durch die Membran fließt.

6. Mikromechanischer Filter nach Anspruch 4, **dadurch gekennzeichnet, dass** die Heizeinrichtung durch einen Heizmäander (23) gebildet wird, der thermisch an die Membran (22) gekoppelt ist.

7. Mikromechanischer Filter nach einem der vorhergehenden Ansprüche, **dadurch gekennzeichnet, dass** die Einrichtung zum Entfernen der Mikropartikel eine Mikropumpe und/oder einen Mikroinjektor (33) umfasst, der eine Strömung parallel zur Oberfläche der Membran (32) erzeugt, welche die Mikropartikel von der Membran (32) löst.

8. Mikromechanischer Filter nach einem der vorhergehenden Ansprüche, **gekennzeichnet durch** eine Vorrichtung (34) zur Vervielfältigung von Bakterien, die von der Oberfläche der Membran (32) entfernt wurden.

9. Mikromechanischer Filter nach einem der vorhergehenden Ansprüche, **gekennzeichnet durch** eine Detektoreinheit zur Detektion der von der Oberfläche der Membran entfernten Mikropartikel.

10. Mikromechanischer Filter nach einem der vorhergehenden Ansprüche, **dadurch gekennzeichnet, dass** die Membran (12; 22; 32; 42) und bevorzugt auch das Substrat (11) aus einkristallinem Silizium gebildet sind.

11. Mikromechanischer Filter nach einem der Ansprüche 1 bis 9, **dadurch gekennzeichnet, dass** die Membran (12; 22; 32; 42) und bevorzugt auch das Substrat (11) aus Siliziumkarbid gebildet ist.

12. Mikromechanischer Filter nach einem der Ansprüche 1 bis 9, **dadurch gekennzeichnet, dass** er aus Metall hergestellt ist, das oxidationsbeständig beschichtet ist.

13. Verfahren zur Herstellung eines mikromechanischer Filters nach einem der Ansprüche 1 bis 12, **dadurch gekennzeichnet, dass** ein Teil eines Substrats (7) porosiziert wird, um eine mit Löchern versehene Schicht (5a; 7a) zu bilden, und ein anderer Teil des Substrats (7) entfernt wird, so dass aus dem Substrat eine Membran herausgearbeitet wird, wobei die Membran durch die mit Löchern versehene Schicht (5a;7a) gebildet wird, und weiterhin eine Einrichtung zum Entfernen von Ablagerungen von einer Oberfläche der Membran ausgestaltet wird, welche eine Aktorstruktur (43) umfasst, die an der Membran (42) befestigt ist, um die Membran (42) zu verformen.

14. Verfahren nach Anspruch 13, **dadurch gekennzeichnet, dass** zunächst das Porosizieren des Substrats (7) von dessen Oberseite her bis zu einer definierten Tiefe durchgeführt wird, und anschließend der andere Teil des Substrats (7) von dessen Unterseite her zumindest teilweise entfernt wird, so dass die poröse Schicht (7a) eine Membran mit durchgehenden Löchern (8a) bildet.

15. Verfahren nach Anspruch 13, **dadurch gekennzeichnet, dass** das Substrat (7) eine untere Substratschicht mit einem darüber angeordneten SOI-Wafer umfasst, und ein Teil der unteren Substratschicht (3) durch Ätzen entfernt wird, wobei die Isolatorschicht (5b) des SOI-Wafers als Ätzstopp verwendet wird.

16. Verfahren nach Anspruch 15, **dadurch gekennzeichnet, dass** nach dem Ätzen der unteren Substratschicht (3) die Isolatorschicht (5b) des SOI-Wafers entfernt wird, und anschließend die Siliziumschicht (5a) des SOI-Wafers porosiziert wird, um die mit durchgehenden Löchern versehene Membran zu bilden.

## Claims

1. Micromechanical filter for microparticles, in particular for pathogenic bacteria and viruses, comprising
a substrate (11), and
a perforated membrane (12; 22; 32; 42), firmly connected to the substrate (11), for filtering microparticles out of a medium as the latter flows through the membrane (12; 22; 32; 42),
**characterized by**
a device (13a, 13b; 23; 33; 43) for removing the microparticles filtered out from the surface of the membrane (12; 22; 32; 42), which device comprises an actuator structure (43) which is fixed so the membrane (42) in order to deform the membrane (42).

2. Micromechanical filter according to Claim 1, **characterized in that** the actuator structure (43) is configured in such a way that it generates wave movements in the form of surface waves in the membrane (42).

3. Micromechanical filter according to Claim 1 or 2, **characterized in that** the actuator structure (43) is formed by one or more FPW structures.

4. Micromechanical filter according to Claim 1, 2 or 3, **characterized in that** the device for removing microparticles comprises a heating device (13a, 13b; 23) for heating the membrane (12; 22), in order to burn the microparticles located on the surface of the membrane (12; 22).

5. Micromechanical filter according to Claim 4, **characterized in that** the heating device is formed by electric contacts (13a, 13b), which are configured in such a way that, when a voltage source is connected, a heating current flows through the membrane.

6. Micromechanical filter according to Claim 4, **characterized in that** the heating device is formed by a heating meander (23), which is coupled thermally to the membrane (22).

7. Micromechanical filter according to one of the preceding claims, **characterized in that** the device for removing the microparticles comprises a micro pump and/or a micro injector (33), which generate/s a flow parallel to the surface of the membrane (32) which detaches the microparticles from the membrane (32).

8. Micromechanical filter according to one of the preceding claims, **characterized by** an apparatus (34) for multiplying bacteria which have been removed from the surface of the membrane (32).

9. Micromechanical filter according to one of the preceding claims, **characterized by** a detector unit for the detection of microparticles removed from the surface of the membrane.

10. Micromechanical filter according to one of the preceding claims, **characterized in that** the membrane (12; 22; 32; 42) and preferably also the substrate (11) is/are formed from monocrystalline silicon.

11. Micromechanical filter according to one of Claims 1 to 9, **characterized in that** the membrane (12; 22; 32; 42) and preferably also the substrate (11) is/are formed from silicon carbide.

12. Micromechanical filter according to one of Claims 1 to 9, **characterized in that** it is produced from metal which is coated so as to resist oxidation.

13. Process for producing a micromechanical filter according to one of Claims 1 to 12, **characterized in that** part of the substrate (7) is made porous in order to form a layer (5a; 7a) provided with holes, and another part of the substrate (7) is removed, so that a membrane is produced out of the substrate, wherein the membrane is formed by the layer (5a; 7a) provided with holes, and, moreover, a device for removing deposits from a surface of the membrane is configured, comprising an actuator structure (43) which is fixed to the membrane (42) in order to deform the membrane (42).

14. Process according to Claim 13, **characterized in that**, firstly, the substrate (7) is made porous from the surface of the latter as far as a defined depth, and then the other part of the substrate (7) is at least partly removed from the underside thereof, so that the porous layer (7a) forms a membrane with continuous holes (8a).

15. Process according to Claim 13, **characterized in that** the substrate (7) comprises a lower substrate layer with an SOI wafer arranged above the said substrate, and part of the lower substrate layer (3) is removed by etching, wherein the isolator layer (5b) of the SOI wafer is used as an etch stop.

16. Process according to Claim 15, **characterized in that**, following the etching of the lower substrate layer (3), the isolator layer (5b) of the SOI wafer is removed, and then the silicon layer (5a) of the SOI wafer is made porous in order to form the membrane provided with continuous holes.

## Revendications

1. Filtre micromécanique pour microparticules, en particulier pour des bactéries et des virus pathogènes, présentant un substrat (11) et une membrane perforée (12 ; 22 ; 32 ; 42), assemblée de manière fixe au substrat (11) pour la séparation par filtration de microparticules d'un milieu lors de l'écoulement au travers de la membrane (12 ; 22 ; 32 ; 42), **caractérisé par** un dispositif (13a, 13b ; 23 ; 33 ; 43) pour éliminer les microparticules séparées par filtration de la surface de la membrane (12 ; 22 ; 32 ; 42), qui comprend une structure d'actionneur (43) qui est fixée à la membrane (42) pour déformer la membrane (42).

2. Filtre micromécanique selon la revendication 1, **caractérisé en ce que** la structure d'actionneur (43) est conçue de manière telle qu'elle génère, dans la membrane (42), des mouvements ondulatoires sous forme d'ondes de surface.

3. Filtre micromécanique selon la revendication 1 ou 2, **caractérisé en ce que** la structure d'actionneur (43) est formée par une ou plusieurs structures FPW (flexural plate wave - onde plate de flexion).

4. Filtre micromécanique selon la revendication 1, 2 ou 3, **caractérisé en ce que** le dispositif pour éliminer les microparticules comprend un dispositif de chauffage (13a, 13b ; 23) pour chauffer la membrane (12 ; 22) en vue de brûler les microparticules se trouvant à la surface de la membrane (12 ; 22).

5. Filtre micromécanique selon la revendication 4, **caractérisé en ce que** le dispositif de chauffage est formé par des contacts électriques (13a, 13b) qui sont conçus de manière telle que lors du raccordement d'une source de tension, un flux chaud s'écoule au travers de la membrane.

6. Filtre micromécanique selon la revendication 4, **caractérisé en ce que** le dispositif de chauffage est formé par un méandre chauffant (23) qui est couplé thermiquement à la membrane (22).

7. Filtre micromécanique selon l'une quelconque des revendications précédentes, **caractérisé en ce que** le dispositif pour éliminer les microparticules comprend une micropompe et/ou un micro-injecteur (33) qui génère un écoulement parallèle à la surface de la membrane (32), qui détache les microparticules de la membrane (32).

8. Filtre micromécanique selon l'une quelconque des revendications précédentes, **caractérisé par** un dispositif (34) pour la multiplication de bactéries qui ont été éliminées de la surface de la membrane (32).

9. Filtre micromécanique selon l'une quelconque des revendications précédentes, **caractérisé par** une unité de détection pour la détection des microparticules éliminées de la membrane.

10. Filtre micromécanique selon l'une quelconque des revendications précédentes, **caractérisé en ce que** la membrane (12 ; 22 ; 32 ; 42) et de préférence également le substrat (11) sont formés en silicium monocristallin.

11. Filtre micromécanique selon l'une quelconque des revendications 1 à 9, **caractérisé en ce que** la membrane (12 ; 22 ; 32 ; 42) et de préférence également le substrat (11) sont formés en carbure de silicium.

12. Filtre micromécanique selon l'une quelconque des revendications 1 à 9, **caractérisé en ce qu'**il est en métal qui est revêtu de manière résistante à l'oxydation.

13. Procédé pour la fabrication d'un filtre micromécanique selon l'une quelconque des revendications 1 à 12, **caractérisé en ce qu'**une partie d'un substrat (7) est rendue poreuse pour former une couche (5a ; 7a) pourvue de trous et une autre partie du substrat (7) est éliminée de telle sorte qu'une membrane est produite à partir du substrat, la membrane étant formée par la couche (5a ;7a) pourvue de trous et, en outre, un dispositif est conçu pour éliminer les dépôts d'une surface de la membrane, qui comprend une structure d'actionneur (43) qui est fixée à la membrane (42) pour déformer la membrane (42).

14. Procédé selon la revendication 13, **caractérisé en ce que** le fait de rendre le substrat (7) poreux est d'abord réalisé à partir de sa face supérieure jusqu'à une profondeur définie et ensuite, l'autre partie du substrat (7) est éliminée à partir de sa face inférieure, au moins partiellement, de manière telle que la couche poreuse (7a) forme une membrane présentant des trous traversants (8a).

15. Procédé selon la revendication 13, **caractérisé en ce que** le substrat (7) comprend une couche inférieure de substrat sur laquelle est disposée une tranche de SOI (silicon on insulator - silicium sur isolant) et une partie de la couche inférieure (3) de substrat est éliminée par gravure, la couche d'isolant (5b) de la tranche de SOI étant utilisée comme arrêt de gravure.

16. Procédé selon la revendication 15, **caractérisé en ce qu'**après la gravure de la couche inférieure (3) de substrat, la couche d'isolant (5b) de la tranche de SOI est éliminée et la couche de silicium (5a) de la tranche de SOI est ensuite rendue poreuse pour former la membrane pourvue de trous traversants.
